# DEMANDE DE BREVET EUROPEEN

(11) **EP 1 702 652 A1**
(43) Date de publication de la demande: **20.09.2006**
(21) Numéro de dépôt: 06290321.6
(22) Date de dépôt: 27.02.2006
(51) Int. Cl.: A61Q 5/10

(54) **Composition cosmétique contenant un composé tribochrome, procédé mettant en oeuvre cette composition et utilisations**

(30) Priorité: 28.02.2005 FR 0502004
(71) Demandeur: L'ORÉAL, 75008 Paris (FR)
(72) Inventeur: Pourille-Grethen, Chrystel, 92110 Clichy (FR); Gourlaouen, Luc, 92600 Asniéres s/Seine (FR)
(74) Mandataire: Casalonga, Axel

(57) **Abrégé**

L'invention a pour objet une composition cosmétique comprenant dans un milieu approprié, au moins un composé tribochrome présentant la propriété de changer de coloration ou de nuance lorsque déposé sur les phanères, les lèvres ou la peau, il subit un changement de conformation, suite à une friction manuelle.

De préférence, le composé tribochrome répond aux formules générales suivantes :

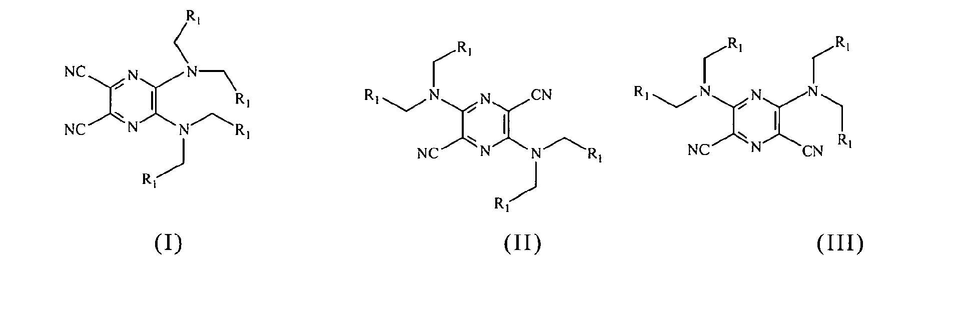

## Description

La présente invention a pour objet une composition cosmétique comprenant au moins un composé tribochrome, ainsi que les procédés et utilisations mettant en oeuvre cette composition.

Dans le domaine cosmétique, en particulier dans le domaine de la coloration capillaire et dans le domaine du maquillage, il existe depuis de nombreuses années un besoin de disposer de compositions colorantes dont la coloration obtenue est susceptible d'être changée ou nuancée de manière simple.

Il est connu de teindre les fibres kératiniques humaines, et en particulier les cheveux, avec des compositions tinctoriales contenant des précurseurs de colorants d'oxydation, appelés généralement bases d'oxydation. Ces bases d'oxydation sont des composés incolores ou faiblement colorés qui, associés à des produits oxydants, donnent naissance à des composés colorés.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Il est aussi connu de teindre les fibres kératiniques humaines par une coloration directe consistant à appliquer sur les fibres kératiniques des colorants directs qui sont des molécules colorées et colorantes ayant une affinité pour lesdites fibres.

Les colorations obtenues sont temporaires ou semi-permanentes, c'est-à-dire qu'elles s'estompent au mieux après seulement 4 à 5 shampooings. Ces colorations présentent l'avantage de ne pas entraîner de dégradation chimique de la kératine.

Ces modes de coloration permettent l'obtention de colorations qui peuvent être chromatiques. Toutefois, elles ne permettent pas à un utilisateur de changer de coloration en un temps court et de manière aisée.

En effet, les changements de coloration dans le domaine capillaire sont généralement générés par des processus de décoloration/coloration utilisant du peroxyde d'hydrogène, qui est un agent agressif pour la fibre kératinique. Ces changements successifs de coloration des fibres kératiniques, telles que les cheveux abîment, ou sensibilisent lesdites fibres. En effet, des écarts de coloration tout au long d'une même fibre kératinique, entre sa pointe et sa racine sont observés sur des cheveux ayant subi plusieurs colorations successives, on parle alors de sélectivité.

De même dans le domaine du maquillage, l'utilisation de produits colorés ou colorants pour embellir le visage, voire d'autres parties du corps, ou pour masquer les imperfections de la peau est largement répandue, comme par exemple l'utilisation de fard, de fond de teint, de poudre pour couvrir la peau, ou l'utilisation de mascara, rouge à lèvres, vernis à ongles, crayons pour maquiller la peau, les lèvres ou les ongles.

La couleur apportée par ces produits (fond de teint ou rouge à lèvres) ne peut être modifiée. En effet, pour obtenir un changement de couleur, il est nécessaire de procéder à un démaquillage, suivi d'un remaquillage utilisant des produits dans les nuances souhaitées.

Il n'existe pas à ce jour de produit de coloration ou de maquillage permettant à partir d'un produit coloré ou colorant existant, de changer sa couleur ou sa nuance de manière rapide et aisée.

Il subsiste par conséquent un besoin de disposer de substances présentant la propriété de changer de couleur ou de nuance de manière uniforme ou non, et de manière rapide et aisée, sans agresser la matière à « recolorer ».

La demanderesse a découvert qu'il était possible de résoudre ce problème en utilisant une composition cosmétique comprenant au moins un composé tribochrome.

Les composés tribochromes sont des composés colorés, présentant la propriété de changer de couleur par simple sollicitation mécanique, de la force d'un frottement manuel. Lorsque ce composé est imprégné ou appliqué sur un support, la couleur du support subira un changement de couleur lors de la sollicitation mécanique.

Cette propriété est connue dans le domaine des encres infalsifiables. La demande internationale WO94/26729 (University College Cardiff Consultants limited) décrit la synthèse et l'utilisation de dérivés de furane dans le domaine des encres infalsifiables.

Des dérivés pyraziniques sont décrits dans la demande internationale WO 03/028684 (L'OREAL). Ce document divulgue leur utilisation en tant que colorants capillaires.

De même, des dérivés de pyrazine sont divulgués dans brevet EP 579 835 (NIPPON SODA). Leur mode de synthèse est décrit et leur utilisation pour la fabrication de films, qui seront utilisés dans la construction de serres, pour modifier la longueur d'onde de la lumière (naturelle ou artificielle) permettant ainsi de favoriser la poussée de plantes.

Aucune de ces deux demandes ne citent de composés spécifiques présentant un caractère tribochrome.

D'autres objets, aspects, caractéristiques, avantages apparaîtront plus clairement à la lecture de la description et des exemples qui suivent.

La présente demande porte sur une composition cosmétique comprenant dans un milieu approprié, au moins un composé tribochrome.

Sans vouloir être lié à une quelconque théorie, la sollicitation mécanique de la force d'un frottement ou d'une friction manuelle induit un changement de conformation du composé tribochrome, qui provoque le changement de couleur.

Préférentiellement, les forces à appliquer pour obtenir l'effet tribochrome souhaité sont comprises entre 0,01 et 50 N et plus particulièrement entre 0,1 et 20 N et encore plus particulièrement entre 1 et 20 N.

Ce changement de couleur est irréversible. Une fois, la conformation de la molécule modifiée, celle-ci ne peut reprendre sa conformation initiale.

La nuance de couleur obtenue est dépendante de la force et de la durée de la sollicitation mécanique exercée sur la matière à recolorer. Plus la sollicitation mécanique sera longue et forte, plus la quantité de composés tribochromes, dont la conformation aura changé sera importante, plus la couleur ou la nuance sera modifiée. Ainsi, il est nécessaire de solliciter mécaniquement la matière à recolorer jusqu'à obtention de la nuance souhaitée.

L'utilisateur pourra ainsi modifier la couleur de manière homogène ou modifier seulement certaines parties de la zone colorée par le composé tribochrome.

Dans le domaine capillaire, le changement de couleur peut être obtenu, par exemple, suite à un brossage de la mèche, ou de la chevelure entière.

Pour les vernis à ongles, le changement de couleur peut être obtenu par action des doigts ou des ongles ou d'un objet adéquat.

Lorsque la composition est appliquée sur la peau, le changement de couleur peut être obtenu par frottement de la peau avec les doigts, un pinceau ou un « blush ».

De préférence, les composés tribochromes utilisables dans les compositions selon l'invention répondent aux formules générales suivantes : 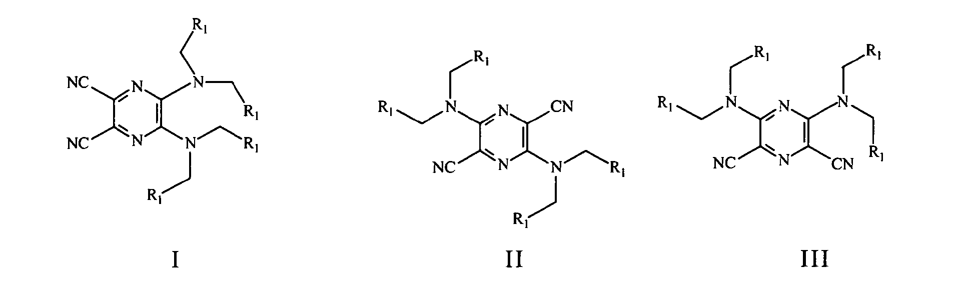 dans lesquelles
R₁ identique ou différent, représente un radical aryle condensé ou non condensé en C₆-C₃₀, éventuellement substitué par un ou plusieurs groupements choisis parmi les atomes d'halogène, tel que F, Cl, I, Br, les groupes alkyle en C₁-C₁₀, hydroxy, alcoxy(C₁₋C₁₀)amino, mono ou dialkyl(C₁-C₁₀)amino, un mono ou dihydroxyalkyl(C₁-C₁₀)amino, alkyl(C₁-C₁₀)hydroxyalkyl(C₁₋C₁₀)amino, mono ou polyhydroxyalkyl(C₁-C₁₀), aryle en C₆-C₃₀, carboxy, alcoxy(C₁-C₁₀)carbonyle, sulfo, acyle en C₂-C₁₀, acyl(C₁₋C₁₀)oxy, aminocarbonyle, nitro, cyano ou uréido.
R₁ pouvant être substitué par un groupe électro-attracteur ou par un groupe électro-donneur, de préférence, le subsituant R₁ représente un radical triméthylphényle, biphényle, naphtalènylméthyle, un chlorophényle ou un bromophényle.
Par « groupe électro-attracteur », on entend un atome d'halogène, un groupe nitro, un groupe cyano ou un groupe -SO₃⁻.
Par « groupe électro-donneur », on entend un groupe alkyle ; alcoxy ; une amine non substituée ou substituée par un groupe alkyle ; un groupe aryle.
De préférence, les substituants R₁ sont identiques.
De manière encore plus préférée, le composé tribochrome est choisi parmi les composés de formules suivantes :

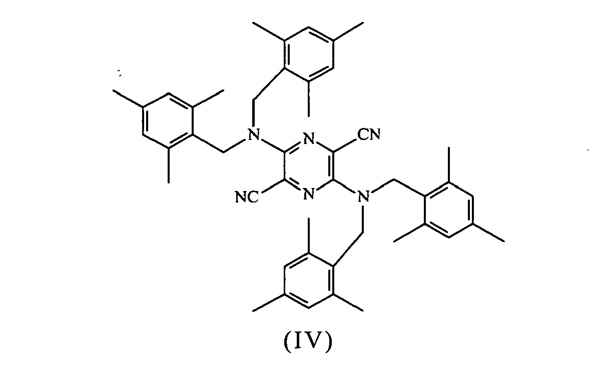

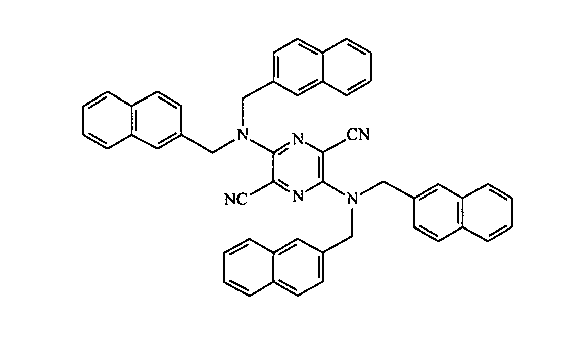

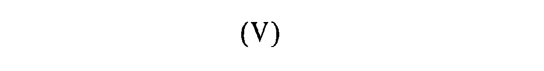

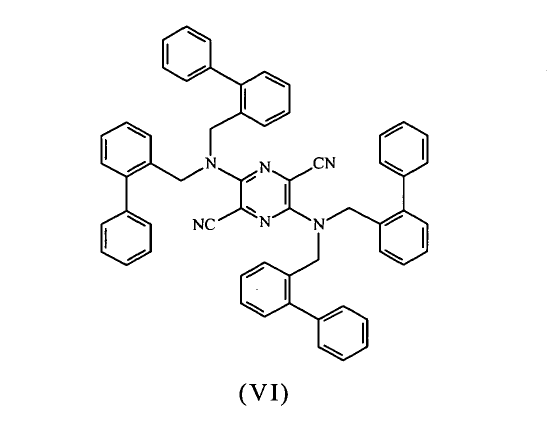

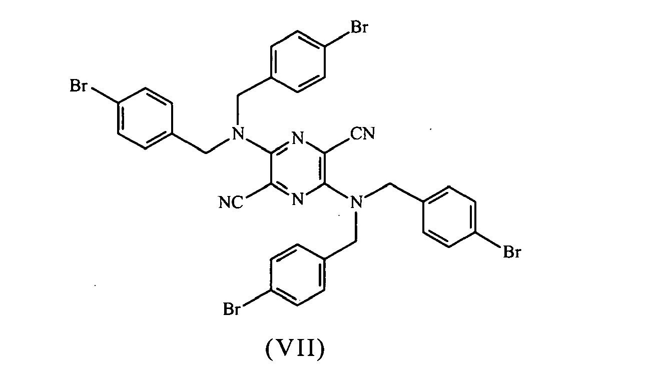

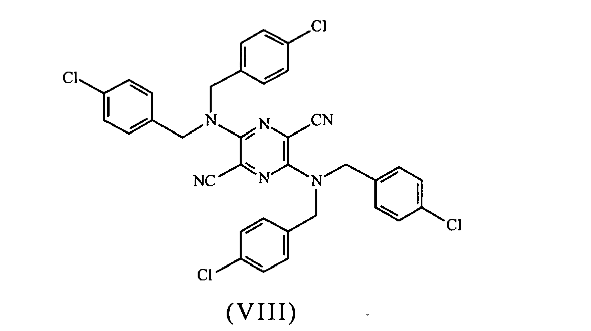

La composition de l'invention comprend entre 0,0001 et 30 %, de préférence de entre 0,01 et 10% en poids du composé tribochrome par rapport au poids total de la composition.

La composition conforme à l'invention, selon une forme de réalisation préférée, et en plus du ou des composés tribochromes comprend au moins un composé choisi parmi les colorants directs, les colorants d'oxydation, les agents tensioactifs et les agents épaississants, les huiles, les cires, les gommes, les pigments, les nacres.

Les colorants directs additionnels peuvent être de nature non ionique, cationique ou anionique, qui peuvent par exemple être choisis parmi les colorants benzéniques rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine, et
- le 1-amino-3-nitro-6-hydroxybenzène.
- le 1-(β-aminoéthyl)amino-2-nitro-4-(β-hydroxyéthyloxy) benzène,
- le 1-(β,γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine, et
- le 1-amino-2-nitro-4-hydroxy-5-méthylbenzène.

La composition conforme à l'invention peut également comprendre, en addition ou en remplacement de ces colorants benzéniques, un ou plusieurs colorants directs additionnels choisis parmi les colorants benzéniques jaunes, jaune-vert, bleus ou violets, les colorants azoïques, les colorants anthraquinoniques, naphtoquinoniques ou benzoquinoniques, les colorants indigoïdes,
ou les colorants dérivés du triarylméthane.

Ces colorants directs additionnels peuvent notamment être des colorants basiques parmi lesquels on peut citer plus particulièrement les colorants connus dans le COLOR INDEX, 3ème édition, sous les dénominations « Basic Brown 16 », « Basic Brown 17 », « Basic Yellow 57 », « Basic Red 76 », « Basic Violet 10 », « Basic Blue 26 » et « Basic Blue 99 », ou des colorants directs acides parmi lesquels on peut plus particulièrement citer les colorants connus dans le COLOR INDEX , 3ème édition, sous les dénominations « Acid Orange 7 », « Acid Orange 24 », « Acid Yellow 36 », « Acid Red 33 », « Acid Red 184 », « Acid Black 2 », « Acid Violet 43 », et « Acid blue 62 », ou encore des colorants directs cationiques tels que ceux décrits dans les demandes de brevet WO 95/01772, WO 95/15144 et EP-A-0 714 954 et dont le contenu fait partie intégrante de la présente invention.

Parmi les colorants directs additionnels benzéniques jaunes et jaune-vert, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène, et
- le 4-(β-hydroxyéthyl)amino-3-nitrobenzamide.

Parmi les colorants directs additionnels benzéniques bleus ou violets, on peut par exemple citer les composés choisis parmi :
- le 1-(β-hydroxyéthyl)amino-4-N,N-bis-(β-hydroxyéthyl)amino-2-nitrobenzène,
- le 1-(γ-hydroxypropyl)amino-4-N,N-bis-(β-hydroxyéthyl)amino-2-nitrobenzène,
- le 1-(β-hydroxyéthyl)amino-4-(N-méthyl, N-β-hydroxyéthyl)amino-2-nitrobenzène,
- le 1-(β-hydroxyéthyl)amino-4-(N-éthyl, N-β-hydroxyéthyl)amino-2-nitrobenzène,
- le 1-(β,γ-dihydroxypropyl)amino-4-(N-éthyl, N-β-hydroxyéthyl)amino-2-nitrobenzène,
- les 2-nitroparaphénylènediamines de formule (IV) suivante : 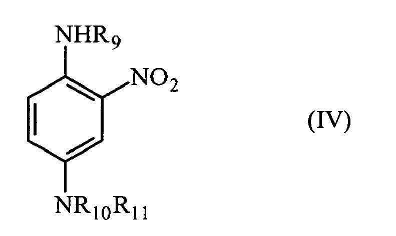
dans laquelle :
R10 représente un radical alkyle en C1-C4, un radical β-hydroxyéthyle ou β-hydroxypropyle ou γ-hydroxypropyle ;
R9 et R11, identiques ou différents, représentent un radical β-hydroxyéthyle, β-hydroxypropyle, γ-hydroxypropyle, ou β,γ-dihydroxypropyle, l'un au moins des radicaux R10, R11 ou R9 représentant un radical γ-hydroxypropyle et R10 et R11 ne pouvant désigner simultanément un radical β-hydroxyéthyle lorsque R9 est un radical γ-hydroxypropyle,
telles que celles décrites dans le brevet français N° 2 692 572.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

La composition de la présente invention peut de plus contenir des bases d'oxydation et des coupleurs classiquement utilisés pour la coloration par oxydation.

A titre d'exemple de bases d'oxydation, on peut citer. les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, les bases d'oxydation et les coupleurs sont chacun présents en une quantité comprise entre 0,001 et 10 % en poids environ du poids total de la composition tinctoriale, de préférence entre 0,005 et 6 %.

Lorsque la composition selon l'invention contient une base d'oxydation et/ou un coupleur, la composition peut alors contenir un agent oxydant. Les agents oxydants classiquement utilisés pour la coloration d'oxydation sont par exemple un oxydant chimique, tel que le peroxyde d'hydrogène, le peroxyde d'urée, les bromates de métaux alcalins, les persels tels que les perborates et persulfates, les peracides. Un oxydant biocatalytique peut également être utilisé, tel que les enzymes oxydases parmi lesquelles on peut citer les peroxydases, les oxydo-réductases à 2 électrons telles que les uricases et les oxygénases à 4 électrons comme les laccases. Cette enzyme générée in situ l'oxydant nécessaire à l'oxydation du précurseur de colorant, à partir d'un substrat approprié et d'oxygène atmosphérique.

Les compositions cosmétiques de la présente invention peuvent en outre comprendre comme indiqué précédemment des huiles, des gommes et/ou des cires.

Les huiles, corps gras liquides à température ambiante, cosmétiquement acceptables peuvent être des huiles hydrocarbonées et/ou siliconées et/ou fluorées. Elles peuvent être d'origine animale, végétale, minérale ou synthétique.

On peut citer en particulier, seule ou en mélange :
- les huiles hydrocarbonées d'origine animale telles que le perhydrosqualène,
- les huiles hydrocarbonées végétales telles que les huiles de tournesol, de maïs, de soja, de courge, de pépins de raisin, d'arachide, d'amande douce, de calophyllum, de palme, de sésame, de noisette, d'abricot, de macadamia, de ricin, d'avocat, de jojoba et de beurre de karité, les triglycérides liquides d'acides gras en C₄-C₁₀, comme les triglycérides des acides heptanoïque ou octanoïque, les triglycérides des acides caprylique/caprique comme ceux vendus par la société Stearineries Dubois ou ceux vendus sous les dénominations Miglyol® 810, 812 et 818 par la société Dynamit Nobel,
- les esters de synthèse, notamment :
   - les esters d'acides gras comme les huiles de formule R3COOR4 dans laquelle R3 représente le reste d'un acide gras supérieur comportant de 7 à 29 atomes de carbone et R4 représente une chaîne hydrocarbonée contenant de 3 à 30 atomes de carbone, comme par exemple le myristate d'isopropyle, le palmitate de 2-éthylhexyle, le stéarate de 2-octyldodécyle, l'éruçate de 2-octyldodécyle et l'isostéarate d'isostéaryle,
   - les esters hydroxylés tels que le lactate d'isostéaryle, l'hydroxystéarate d'octyle, l'hydroxystéarate d'octyldodécyle, le malate de diisostéaryle et le citrate de triisocétyle,
   - les esters de polyols comme le dioctanoate de propylèneglycol, le diheptanoate de néopentylglycol, le diisononanoate de diéthylèneglycol et les esters du pentaérythritol,
- les alcools gras ayant de 12 à 26 atomes de carbone comme l'octyldodécanol, le 2-butyloctanol, le 2-hexyldécanol, le 2-undécylpentadécanol et l'alcool oléylique,
- les huiles hydrocarbonées partiellement fluorées et/ou siliconées,
- les huiles siliconées telles que les polydiméthylsiloxanes, volatiles ou non, linéaires ou cycliques, les alkyldiméthicones, les silicones modifiées par des groupements aliphatiques et/ou aromatiques éventuellement fluorés, ou par des groupements fonctionnels tels que des groupements hydroxyles, thiols et/ou amine, les huiles siliconées phénylées telles que les polyphénylméthylsiloxanes ou les phényltriméthicones.

Les huiles employées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25 °C , supérieure à 0 Pa, en particulier allant de 0,13 à 40 000 Pa. On peut citer notamment les huiles siliconées volatiles telles que les silicones volatiles cycliques ou linéaires, et les cyclocopolymères. On peut également citer les huiles volatiles hydrocarbonées telles que les isoparaffines, et les huiles fluorées volatiles.

Parmi les gommes et/ou cires cosmétiques acceptables susceptibles d'être utilisées on peut citer :
- les gommes de silicone,
- les cires d'origine animale, végétale, minérale ou synthétique telles que les cires microcristallines, la paraffine, le pétrolatum, la vaseline, l'ozokérite, la cire de lignite, la cire d'abeille, la lanoline et ses dérivés, la cire de Candellila, la cire d'Ouricury, la cire de Carnauba, la cire du Japon, le beurre de cacao, la cire de fibres de liège, la cire de canne à sucre, les huiles hydrogénées concrètes à 25°C, les esters gras et glycérides concrets à température ambiante, les cires de polyéthylène et les cires obtenues par synthèse de Fischer-Tropsch,
- les cires de silicone et
- les cires fluorées.

Les compositions cosmétiques de la présente invention peuvent contenir en outre un ou plusieurs agents épaississants, un ou plusieurs polymères filmogènes et/ou un ou plusieurs agents plastifiants.

Une phase particulaire constituée de pigments et/ou de nacres peut également être présente dans les compositions cosmétiques de la présente invention.

Par pigments, il faut comprendre des particules blanches ou colorées, minérales ou organiques destinées à colorer ou à opacifier la composition. On peut citer par exemple les dioxydes de titane, de zirconium ou de cérium, les oxyde de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que les poudres d'argent ou d'aluminium. On peut également citer certaines laques tels que les sels de calcium, de baryum, d'aluminium ou de zirconium. Ces pigments sont généralement présents à raison de 0 à 15 % en poids et de préférence à raison de 8 à 10 % de la composition finale.

Par nacres, il faut comprendre des particules irisées qui réfléchissent la lumière. On peut citer par exemple la nacre naturelle, le mica recouvert d'oxyde de titane, d'oxyde de fer, de pigments naturels ou d'oxychlorure de bismuth ainsi que le mica titane coloré.

Les nacres sont généralement présentes à raison de 0 à 20 % en poids, de préférence à raison de 8 à 15 % en poids de la composition cosmétique finale.

Les compositions selon l'invention peuvent également comprendre des agents épaississants et/ou des tensio-actifs. Les agents épaississants peuvent être d'origine minérale (silice) ou organique. Les agents épaississants organiques encore appelé "agents d'ajustement de la rhéologie" sont de préférence polymériques.

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose.), la gomme de guar et ses dérivés (hydroxypropylguar.), les gommes d'origine microbienne (gomme de xanthane, gomme de scléroglucane.), les homopolymères réticulés d'acide acrylique ou d'acide acrylamidopropanesulfonique et les polymères associatifs tels que décrits ci-dessous.

Leur concentration en poids dans la composition selon l'invention colorante peut varier d'environ 0,01 à 10%, de préférence de 0,1 à 5% du poids total de la composition.

Les polymères associatifs utilisables selon l'invention sont des polymères hydrosolubles capables, dans un milieu aqueux, de s'associer réversiblement entre eux ou avec d'autres molécules.

Leur structure chimique comprend des zones hydrophiles, et des zones hydrophobes caractérisées par au moins une chaîne grasse.

Les polymères associatifs utilisables selon l'invention peuvent être de type anionique, cationique, amphotère et de préférence non ionique.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XV) suivante :

   CH₂ = C R' CH₂ O Bₙ R (XV)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XV) plus particulièrement préféré est un motif dans lequel R' désigne H, n est égal à 10, et R désigne un radical stéaryl (C₁₈).
   Des polymères associatifs anioniques de ce type sont décrits et préparés, selon un procédé de polymérisation en émulsion, dans le brevet EP-0 216 479.
   Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (XV), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC80® et SALCARE SC90® qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (XVI) suivante : 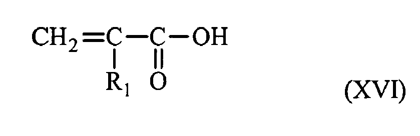 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (XVII) suivante 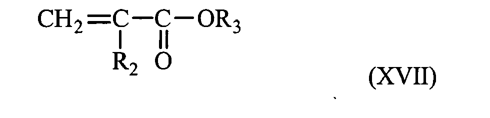 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères associatifs anioniques, on utilisera plus particulièrement des polymères formés à partir d'un mélange de monomères comprenant :
   (i) essentiellement de l'acide acrylique,
   (ii) un ester de formule (XVII) décrite ci-dessus et dans laquelle R₂ désigne H ou CH₃, R₃ désignant un radical alkyle ayant de 12 à 22 atomes de carbone, et
   (iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.
   Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

Parmi les polymères associatifs de type cationique, on peut citer :
- (I) les polyuréthanes associatifs cationiques dont la famille a été décrite par la demanderesse dans la demande de brevet français N° 0009609; elle peut être représentée par la formule générale (XVIII) suivante :

   R-X-(P)n-[L-(Y)m]r-L'-(P')p-X'-R' (XVIII)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
      r est un nombre entier compris entre 1 et 100, de préférence entre 1 et 50 et en particulier entre 1 et 25,
      n, m, et p valent chacun indépendamment des autres entre 0 et 1000 ;
      la molécule contenant au moins une fonction amine protonée
   ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (XVIII) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (XVIII) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (Ia) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 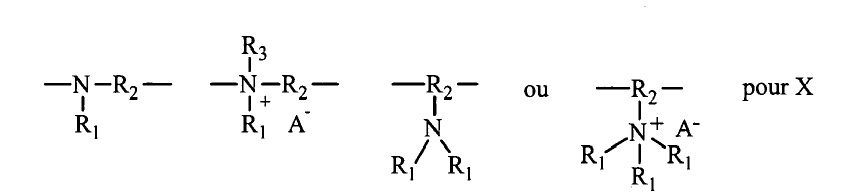 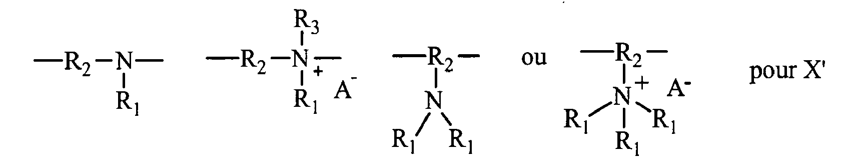 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 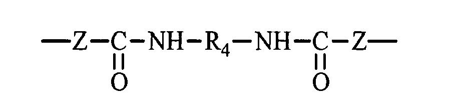 dans laquelle :
Z représente ―O-, -S- ou ―NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 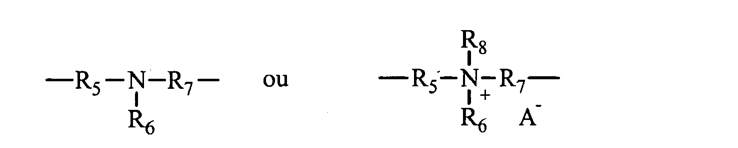 ou 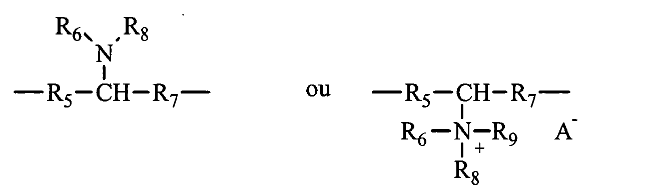 ou 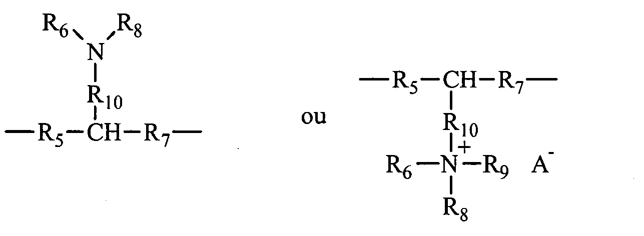 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (XVIII) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (XVIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)n-ZH,

ou

HZ-(P')p-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (XVIII) est un diisocyanate correspondant à la formule :

O=C=N-R₄-N=C=O

dans laquelle R₄ est défini plus haut.

A titre d'exemple, on peut citer le méthylènediphényldiisocyanate, le méthylènecyclohexanediisocyanate, l'isophoronediisocyanate, le toluènediisocyanate, le naphtalènediisocyanate, le butanediisocyanate, l'hexanediisocyanate.

Un troisième composé entrant dans la préparation du polyuréthane de formule (XVIII) est un composé hydrophobe destiné à former les groupes hydrophobes terminaux du polymère de formule (XVIII).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.

Le groupe hydrophobe du polyuréthane de formule (XVIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (XVIII) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.

Les dérivés de cellulose quaternisée sont en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C12) et CRODACEL QS® (alkyle en C₁₈) commercialisés par la société CRODA.

### Polymères associatifs amphotères

Les polymères associatifs amphotères sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
1) au moins un monomère de formule (XIX) ou (XX) : 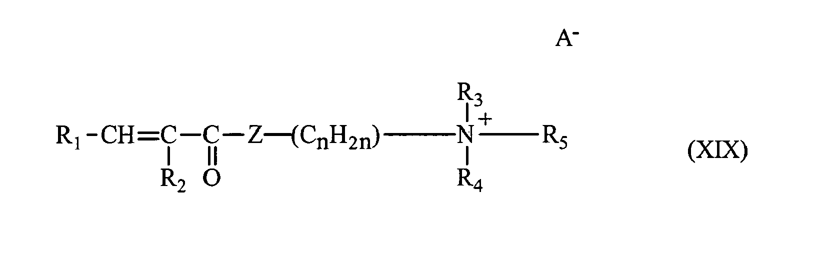 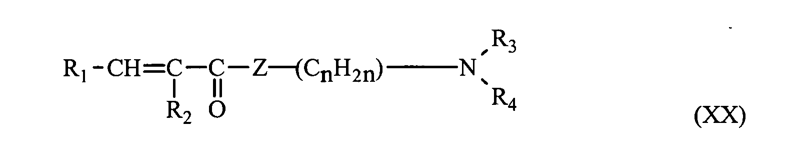 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure;
2) au moins un monomère de formule (XXI)

   R₆―CH=CR₇―COOH (XXI)

   dans laquelle, R₆ et R₇, identiques ou différents, représentent un atome d'hydrogène ou un radical méthyle;
   et
3) au moins un monomère de formule (XXII) :

   R₆―CH =CR₇ ―COXR₈ (XXII)

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
   l'un au moins des monomères de formule (XIX), (XX) ou (XXII) comportant au moins une chaîne grasse.

Les monomères de formule (XIX) et (XX) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
   ces monomères étant éventuellement quaternisés, par exemple par un halogénure d'alkyle en C₁-C₄ ou un sulfate de dialkyle en C₁-C₄.

Plus particulièrement, le monomère de formule (XIX) est choisi parmi le chlorure d'acrylamidopropyl triméthyl ammonium et le chlorure de méthacrylamidopropyl triméthyl ammonium.

Les monomères de formule (XXI) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XXI) est l'acide acrylique.

Les monomères de formule (XXII) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 % moles du monomère comportant une chaîne grasse (monomère de formule (XIX), (XX) ou (XXII)), et de préférence de 1,5 à 6% moles.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂₋₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

La composition peut également contenir des charges.

On entend par « charges » dans la présente invention des particules incolores ou blanches, minérales ou synthétiques, lamellaires ou non, destinées à donner du corps ou de la rigidité à la composition et/ou de conférer au maquillage de la douceur, de la matité et de l'uniformité. Les charges utilisables dans les compositions cosmétiques de la présente invention sont choisies par exemple parmi le talc, le mica, la silice, le kaolin, les poudres de Nylon et de polyéthylène, le Teflon®, l'amidon, le nitrure de bore, les microsphères de polymères telles que Expancel® de la société Nobel Industrie ou Polytrap® de la société Dow Corning, les microbilles de résine de silicone telles que Tospearls® de la société Toshiba, le carbonate de calcium précipité, le carbonate ou hydrocarbonate de magnésium, les savons métalliques dérivés d'acides carboxyliques en C₈-C₂₂.

Les charges sont généralement utilisées à raison de 0 à 80 % en poids, de préférence à raison de 5 à 15 % en poids rapporté au poids final de la composition cosmétique.

Le milieu approprié pour les compositions cosmétiques est constitué par de l'eau, des cétones telles que la méthyléthylcétone, la méthylisobutylcétone, la diisobutylcétone, l'isophorone, la cyclohexanone ou l'acétone, les alcools inférieurs, tels que l'éthanol, l'isopropanol, le diacétone-alcool, le 2-butoxyéthanol ou le cyclohexanol, les polyols tels que le propylèneglycol ou le pentylèneglycol ou les polyéthylènes glycols, les éthers d'alkylèneglycol tels l'éther monométhylique de propylèneglycol, l'acétate de l'éther monométhylique de propylèneglycol ou l'éther de dipropylèneglycol, les acétates d'alkyle en C₂-C₇ tels que l'acétate de méthyle, l'acétate d'éthyle, l'acétate de propyle, l'acétate de butyle ou l'acétate d'isopentyle, les éthers tels que l'éther diéthylique, l'éther diméthylique ou le diclhorodiéthyléther, et les huiles volatiles telles que les huiles siliconées volatiles cycliques ou linéaires, les huiles volatiles hydrocarbonées de synthèse ou encore les huiles fluorées ou leurs mélanges.

Ces liquides à caractère solvant peuvent être présents dans des proportions de préférence comprises entre 1 et 40 % en poids environ par rapport au poids total de la composition tinctoriale, et encore plus préférentiellement entre 5 et 30 % en poids environ.

La composition peut comprendre un certain nombre d'additifs usuellement utilisés dans le domaine cosmétique tels que des agents antioxydants, des parfums, des conservateurs, des actifs cosmétique lipophiles ou hydrophiles, des agents hydratants, des vitamines, des acides gras essentiels, des sphingolipides, des agents autobronzants, des filtres sôlaires, des agents anti-mousse, des agents anti-radicalaires, des polymères anioniques, cationiques, non-ioniques, amphotères, zwittérioniques ou leurs mélanges autres que les polymères épaississants décrits ci-dessus, des agents de pénétration, des agents séquestrants, des tampons, des agents dispersants, des agents de conditionnement autres que ceux cités ci-dessus, des polymères cationiques, des chitosanes et dérivés, des céramides, des agents conservateurs, des acides aminés tels que arginine, cystéine, glycine ou taurine, des agents opacifiants.

Ces adjuvants ci-dessus sont en général présents en quantité comprise pour chacun d'eux entre 0,01 et 20 % en poids par rapport au poids de la composition.

De préférence, les compositions de l'invention contiennent au moins un agent tensioactif et/ou au moins un agent épaississant.

Bien entendu l'homme du métier veillera à choisir les éventuels composés complémentaires de manière à ce que les propriétés avantageuses de la composition selon l'invention ne soient pas, ou pratiquement pas altérées par l'adjonction envisagée.

Le pH de la composition de coloration conforme à l'invention est généralement compris entre 3 et 12 environ, et de préférence entre 5 et 11 environ.

Il peut être ajusté à la valeur désirée au moyen d'agents acidifiants ou alcalinisants habituellement utilisés en coloration ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 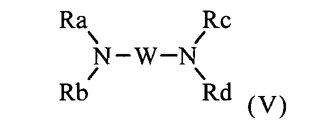 dans laquelle W est un reste propylène éventuellement substitué par un groupement hydroxyle ou un radical alkyle en C₁₋C₄; Ra, Rb, Rc et Rd, identiques ou différents, représentent un atome d'hydrogène, un radical alkyle en C₁-C₄ ou hydroxyalkyle en C₁-C₄.

Les compositions cosmétiques de la présente invention peuvent se présenter sous n'importe quelle forme habituellement rencontrée dans le domaine cosmétique, c'est-à-dire sous forme d'une lotion, d'une suspension, d'une dispersion, d'une solution organique, aqueuse ou hydroalcoolique éventuellement épaissie ou gélifiée, d'une mousse, d'un spray, d'une émulsion huile-dans-eau, eau-dans-huile ou multiple, d'une poudre libre, compacte ou coulée, d'un solide ou d'une pâte anhydre, d'une crème.

Il peut s'agir plus particulièrement d'un produit de soin, d'hygiène et/ou de maquillage. Des modes de réalisation préférés des compositions cosmétiques de la présente invention sont représentés par les compositions capillaires telle qu'une composition tinctoriale, les vernis à ongles et les compositions de maquillage du visage, du corps ou des phanères (ongles, cils, sourcils, cheveux), telles que fard à paupières ou à joues, eye-liner, mascara, poudre libre ou compacte, fond de teint, crème teintée, rouge à lèvres, stick anti-cernes etc.

Un second objet consiste en un procédé de changement de coloration ou de nuance de la peau, des fibres kératiniques, des lèvres ou des phanères mettant en oeuvre la composition précédemment décrite. Dans un premier temps, ladite composition est appliquée sur la peau, les lèvres, les phanères éventuellement en présence d'un agent oxydant. Ensuite, la peau, les lèvres, les phanères, subissent une sollicitation mécanique, de préférence une friction manuelle, de manière à obtenir le changement de coloration
ou de nuance désirée. La durée et la force de frottement sont fonction de la nuance souhaitée.

Un autre objet de l'invention est un dispositif à plusieurs compartiments ou "kit", de préférence à 2 compartiments, pour la teinture de fibres kératiniques, et plus particulièrement les cheveux, dans lequel un premier compartiment renferme la composition tinctoriale de l'invention et un deuxième compartiment renferme un additif cosmétique tel que défini ci-dessus. Ce dispositif peut être équipé d'un moyen permettant de délivrer sur les cheveux le mélange souhaité, tel que les dispositifs décrits dans le brevet FR-2 586 913 au nom de la demanderesse.

L'invention porte également sur l'utilisation de la composition telle que définie ci-dessus en coloration capillaire et pour le maquillage pour la peau, les lèvres ou les phanères en particulier pour permettre des changements de teintes.

Les exemples qui suivent illustrent l'invention sans en limiter la portée.

### Exemples:

### Exemple 1 :

Le colorant de formule (IV) est incorporé dans la formulation suivante :

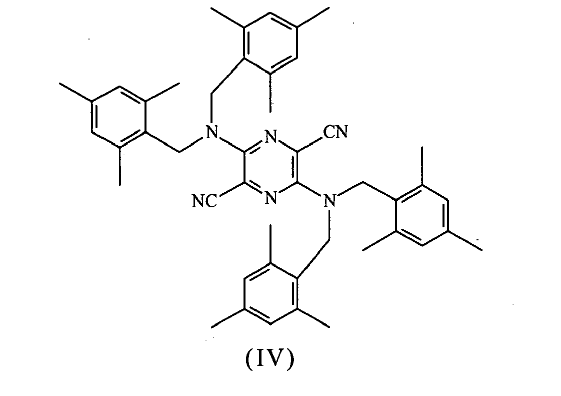

La formulation suivante a été préparée :

| Composé | Quantité |
|---|---|
| Colorant de formule (IV) | 10⁻³ mole % |
| Cyclopentasiloxane | Qsp 100g |

La formulation est appliquée sur la peau, les lèvres ou les phanères, puis la silicone volatile est laissée évaporer. A ce stade, on obtient une coloration jaune, qui par simple frottement mécanique devient orange.

### Exemple 2 :

Le colorant de formule (V) est incorporé dans la formulation suivante :

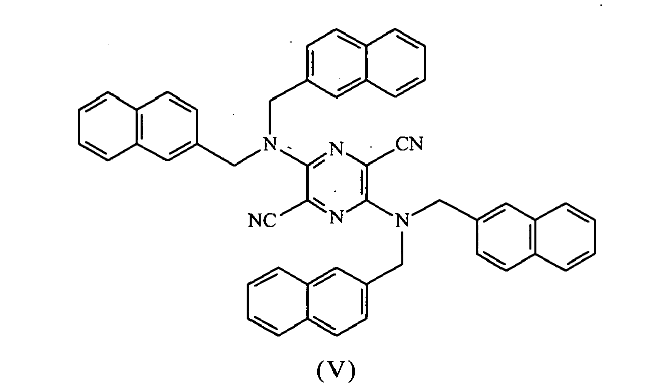

La formulation suivante a été préparée :

| Composé | Quantité |
|---|---|
| Colorant de formule (V) | 10⁻³ mole % |
| Cyclopentasiloxane | Qsp 100g |

La formulation est appliquée sur la peau, les lèvres ou les phanères, puis la silicone volatile est laissée évaporer. A ce stade, on obtient une coloration jaune, qui par simple frottement mécanique devient orange.

### Exemple 3 :

Le colorant de formule (VI) est incorporé dans la formulation suivante :

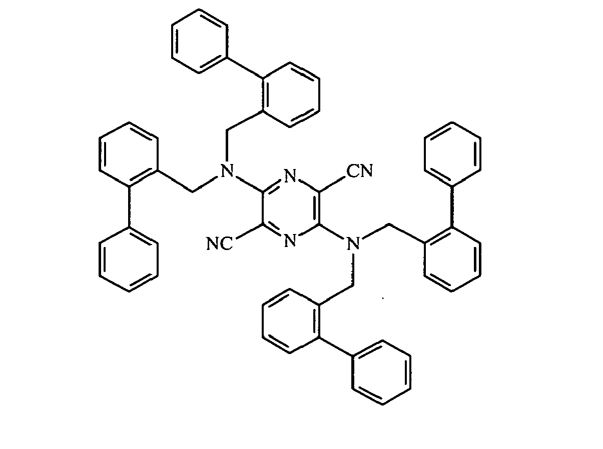

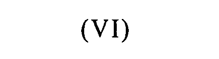

La formulation suivante a été préparée :

| Composé | Quantité |
|---|---|
| Colorant de formule (VI) | 10⁻³ mole % |
| Cyclopentasiloxane | Qsp 100g |

La formulation est appliquée sur la peau, les lèvres ou les phanères, puis la silicone volatile est laissée évaporer. A ce stade, on obtient une coloration jaune, qui par simple frottement mécanique devient orange.

### Exemple 4 :

Le colorant de formule (VII) est incorporé dans la formulation suivante :

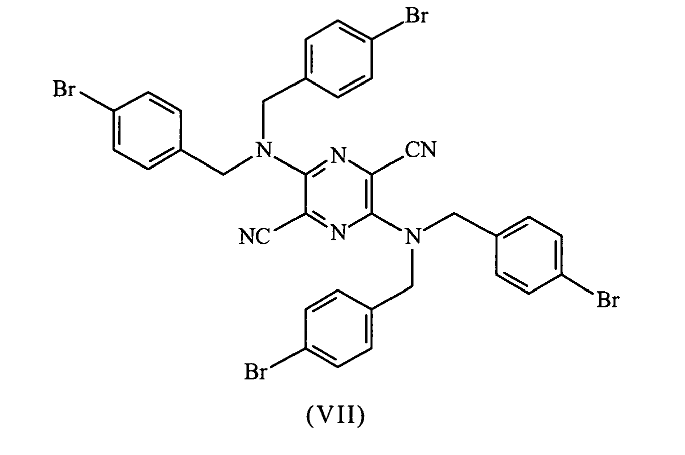

La formulation suivante a été préparée :

| Composé | Quantité |
|---|---|
| Colorant de formule (VII) | 10⁻³ mole % |
| Cyclopentasiloxane | Qsp 100g |

La formulation est appliquée sur la peau, les lèvres ou les phanères, puis la silicone volatile est laissée évaporer. A ce stade, on obtient une coloration jaune, qui par simple frottement mécanique devient orange.

### Exemple 5 :

Le colorant de formule (VIII) est incorporé dans la formulation suivante :

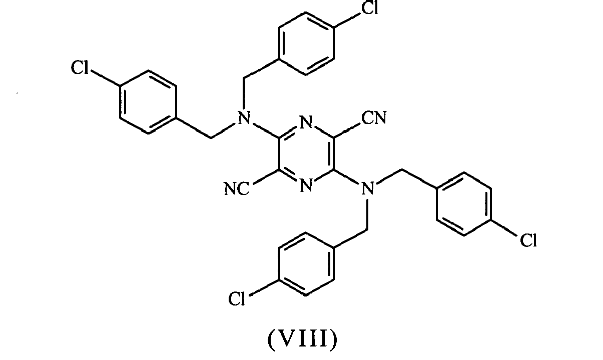

La formulation suivante a été préparée :

| Composé | Quantité |
|---|---|
| Colorant de formule (VIII) | 10⁻³ mole % |
| Cyclopentasiloxane | Qsp 100g |

La formulation est appliquée sur la peau, les lèvres ou les phanères, puis la silicone volatile est laissée évaporer. A ce stade, on obtient une coloration jaune, qui par simple frottement mécanique devient orange.

### Exemple 6 :

La formulation suivante a été préparée :

| Composé | Quantité |
|---|---|
| Colorant de formule (IV) | 10⁻² mole % |
| Alcool benzylique | 5g |
| Ethanol | 20g |
| Lauryléther sulfate à 2 moles d'OE | 2g MA |
| Eau | Qsp 100g |

La formulation est appliquée sur les cheveux. Après séchage, on obtient une coloration jaune qui devient orange par simple frottement mécanique.

## Revendications

1. Composition cosmétique comprenant dans un milieu approprié, au moins un composé tribochrome.

2. Composition cosmétique selon la revendication 1, **caractérisé en ce qu'**elle contient au moins un composé choisi parmi les colorants directs, les colorants d'oxydation, les agents tensio-actifs, les agents épaississants, les huiles, les cires, les gommes, les pigments, les nacres.

3. Composition cosmétique selon l'une des revendications 1 ou 2, **caractérisé en ce que** le composé tribochrome répond aux formules générales suivantes : 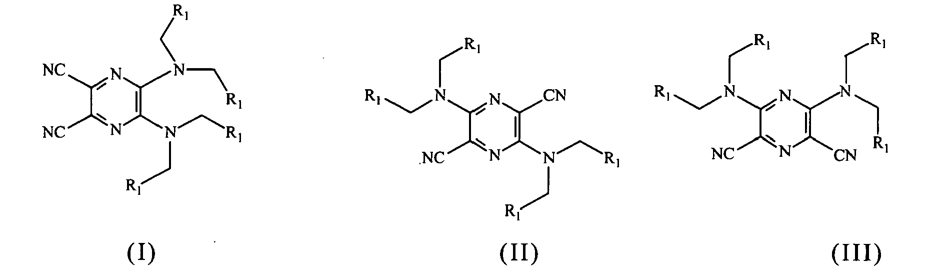 dans lesquelles
R₁ identique ou différent, représente un radical aryle condensé ou non condensé en C₆-C₃₀, éventuellement substitué par un ou plusieurs groupements choisis parmi les atomes d'halogène, les groupes alkyle en C₁-C₁₀, hydroxy, alcoxy(C₁-C₁₀)amino, mono ou dialkyl(C₁-C₁₀)amino, mono ou dihydroxyalkyl(C₁-C₁₀)amino, alkyl(C₁-C₁₀)hydroxyalkyl(C₁-C₁₀)amino, mono ou polyhydroxyalkyl(C₁-C₁₀), aryle en C₆-C₃₀, carboxy, un alcoxy(C₁₋C₁₀)carbonyle, sulfo, acyle en C₂-C₁₀, acyl(C₁-C₁₀)oxy, aminocarbonyle, nitro, cyano ou uréido, R₁ pouvant être substitué par un groupe électro-attracteur ou par un groupe électro-donneur.

4. Composition cosmétique selon la revendication 3, **caractérisé en ce que** les subsituants R₁ sont identiques.

5. Composition cosmétique selon la revendication 4, **caractérisé en ce que** les subsituants R₁ sont identiques et représentent un triméthylphényle, un biphényle, un naphtalènylméthyle, un chlorophényle ou un bromophényle.

6. Composition selon la revendication 3, **caractérisée par le fait que** le groupe électro-attracteur est un atome d'halogène, un groupe nitro, un groupe cyano ou un groupe -SO₃⁻.

7. Composition selon la revendication 3, **caractérisée par le fait que** le groupe électro-donneur est un groupe alkyle, alcoxy, une amine non substituée ou substituée par un groupe alkyle, un groupe aryle.

8. Composition selon l'une quelconque des revendications précédentes, **caractérisée par le fait que** le composé tribochrome est choisi parmi les composés de formules suivantes :

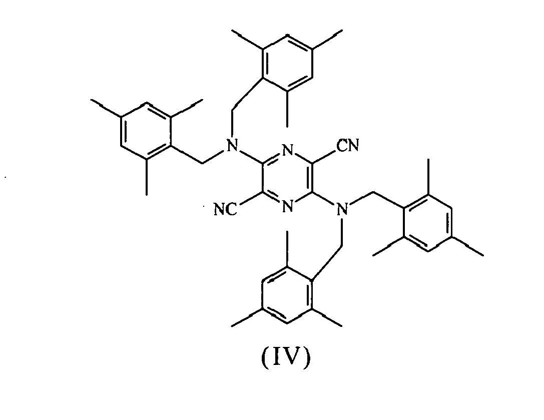

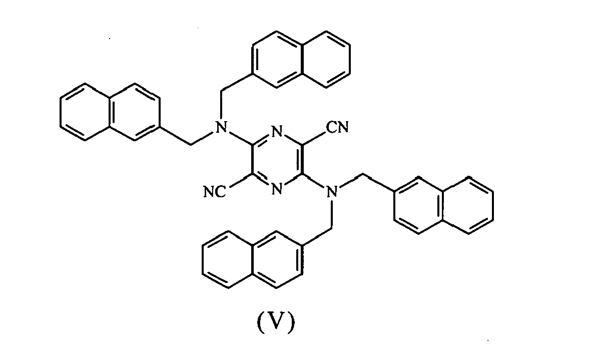

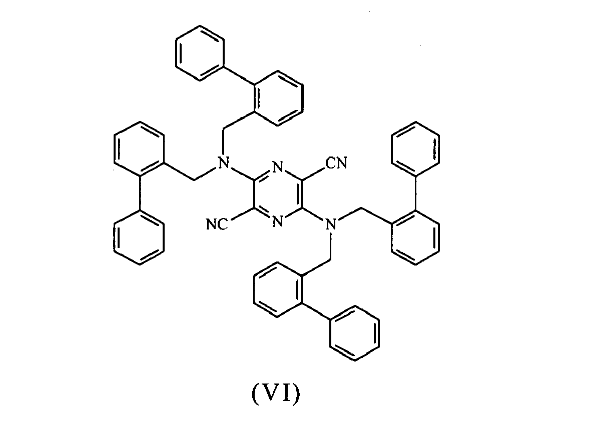

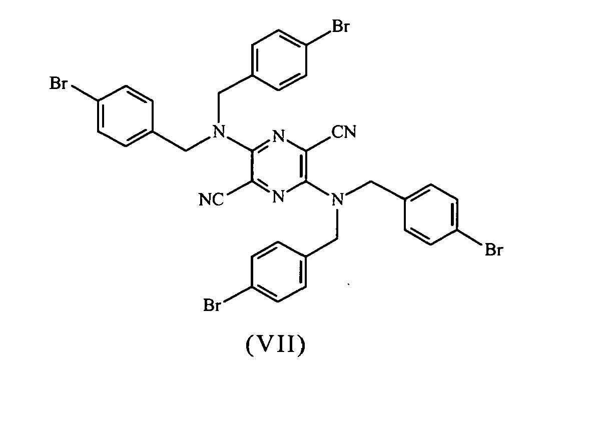

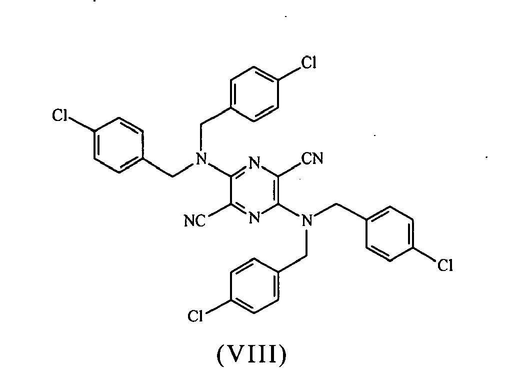

9. Composition selon l'une quelconque des revendications précédentes, **caractérisée en ce qu'**elle comprend entre 0,0001 et 30 %, de préférence de 0,01 à 10% en poids du composé tribochrome défini à l'une quelconque des revendication 1 à 8 par rapport au poids total de la composition.

10. Composition selon l'une quelconque des revendications 2 à 9, **caractérisée en ce qu'**elle contient au moins un colorant direct choisi parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

11. Composition selon la revendication 10, **caractérisée en ce que** la concentration du ou des colorants directs est comprise entre 0,001 et 20% en poids environ du poids total de la composition, et de préférence entre 0,005 et 10% en poids.

12. Composition selon l'une quelconque des revendications 2 à 11, **caractérisée en ce qu'**elle contient au moins une base d'oxydation choisie parmi les paraphénylènediamines, les bis-phénylalkylènediamines, les para-aminophénols, les ortho-aminophénols, les bases hétérocycliques et leurs sels d'addition.

13. Composition selon l'une quelconque des revendications 2 à 12, **caractérisée en ce qu'**elle contient au moins un coupleur choisi parmi les coupleurs métaphénylènediamines, les coupleurs méta-aminophénols, les coupleurs métadiphénols, les coupleurs naphtaléniques, les coupleurs hétérocycliques et leurs sels d'addition.

14. Composition selon la revendication 12 ou 13, **caractérisée en ce que** les bases d'oxydation et les coupleurs sont chacun présents en une quantité comprise entre 0,001 et 10 % en poids environ du poids total de la composition tinctoriale, de préférence entre 0,005 et 6 %.

15. Composition selon l'une quelconque des revendications 12 à 14, **caractérisée en ce qu'**elle contient au moins un agent oxydant.

16. Composition selon l'une quelconque des revendications 2 à 15, **caractérisée en ce qu'**elle comprend une phase grasse composée de corps gras, d'huiles ou cires à température ambiante, d'origine animale, végétale, minérale ou synthétique.

17. Composition selon la revendication 16, **caractérisé en ce que** l'huile est une huile volatile de préférence siliconée.

18. Composition selon l'une quelconque des revendications 2à 17, **caractérisée en ce qu'**elle comprend un ou plusieurs agents épaississants, minéraux ou organiques.

19. Composition selon la revendication 18, **caractérisé en ce que** l'agent épaississant organique est un polymère.

20. Composition selon l'une quelconque des revendications 2 à 19, **caractérisé en ce que** l'agent tensio-actif est anionique, non-ionique, amphotère ou cationique.

21. Composition selon l'une quelconque des revendications précédentes, **caractérisée en ce que** le milieu approprié pour les compositions cosmétiques comprend un ou plusieurs additifs tels que des agents antioxydants, des parfums, des conservateurs, des actifs cosmétique lipophiles ou hydrophiles, des agents hydratants, des vitamines, des acides gras essentiels, des sphingolipides, des agents autobronzants, des filtres solaires, des agents anti-mousse, des agents anti-radicalaires, des polymères anioniques, cationiques, non-ioniques, amphotères, zwittérioniques ou leurs mélanges, des agents de pénétration, des agents séquestrants, des tampons, des agents dispersants, des agents de conditionnement, des polymères cationiques, des chitosanes et dérivés, des céramides, des agents conservateurs, des acides aminés tels que arginine, cystéine, glycine ou taurine, des agents opacifiants.

22. Composition selon l'une quelconque des revendications précédentes, **caractérisée en ce que** le milieu approprié pour les compositions cosmétiques comprend un ou plusieurs solvants appropriés choisis parmi l'eau, les cétones, les alcools, les polyols, les éthers d'alkylèneglycol, les acétates d'alkyles en C₂-C₇, les éthers, les aldéhydes.

23. Composition selon l'une quelconque des revendications précédentes, **caractérisée en ce qu'**elle se présente sous forme d'une lotion, d'une suspension, d'une dispersion, d'une solution organique, aqueuse ou hydroalcoolique éventuellement épaissie ou gélifiée, d'une mousse, d'un spray, d'une émulsion huile-dans-eau, eau-dans-huile ou multiple, d'une poudre libre, compacte ou coulée, d'un solide ou d'une pâte anhydre, d'une crème.

24. Composition selon l'une quelconque des revendications précédentes, **caractérisée par le fait qu'**il s'agit d'une composition tinctoriale pour les fibres kératiniques.

25. Composition selon l'une quelconque des revendications précédentes, **caractérisée par le fait qu'**il s'agit d'un vernis à ongle.

26. Composition selon l'une quelconque des revendications précédentes, **caractérisée par le fait qu'**il s'agit d'une composition de maquillage.

27. Procédé de changement de coloration ou de nuance de la peau, des fibres kératiniques, des lèvres ou des phanères, **caractérisé par le fait qu'**il comprend les étapes suivantes :
- la composition définie à l'une quelconques des revendications 1 à 26 est appliquée sur la peau, les lèvres ou les phanères, puis
- la peau, les lèvres, les phanères subissent une sollicitation mécanique, de préférence une friction manuelle, de manière à obtenir le changement de coloration ou de nuance désiré, la durée et la force de frottement étant fonction de la nuance souhaitée.

28. Procédé selon la revendication 27, **caractérisé en ce que** la force de frottement est comprise entre 0,01 et 50 N de préférence entre 0,1 et 20 N et encore plus préférentiellement entre 1 et 20 N.

29. Dispositif à deux compartiments, ou "kit", pour la teinture des fibres kératiniques humaines, et plus particulièrement des cheveux, dans lequel un premier compartiment contient une composition telle que définie à l'une quelconque des revendications 1 à 26 et un deuxième compartiment contient un additif cosmétique.

30. Utilisation de la composition telle que définie à l'une quelconque des revendications 1 à 26 en coloration capillaire.

31. Utilisation de la composition telle que définie à l'une quelconque des revendications 1 à 26 en maquillage pour la peau, les lèvres ou les phanères.

32. Utilisation selon les revendications 30 ou 31 permettant un changement de teinte du substrat.
